Europäisches Patentamt

**European Patent Office**

Office européen des brevets

(11) Veröffentlichungsnummer: **0 031 100**
**B1**

(12) **EUROPÄISCHE PATENTSCHRIFT**

(45) Veröffentlichungstag der Patentschrift:
**06.10.82**

(51) Int. Cl.³: **C 07 C 69/67,** C 07 C 67/38

(21) Anmeldenummer: **80107852.8**

(22) Anmeldetag: **12.12.80**

(54) Verfahren zur Herstellung von Formylvaleriansäureestern.

(30) Priorität: **22.12.79 DE 2951950**

(43) Veröffentlichungstag der Anmeldung:
**01.07.81 Patentblatt 81/26**

(45) Bekanntmachung des Hinweises auf die Patenterteilung:
**06.10.82 Patentblatt 82/40**

(84) Benannte Vertragsstaaten:
**BE CH DE FR GB IT LI NL**

(56) Entgegenhaltungen:
**US-A-3 445 505**

(73) Patentinhaber: **BASF Aktiengesellschaft,
Carl-Bosch-Strasse 38, D-6700 Ludwigshafen (DE)**

(72) Erfinder: **Kummer, Rudolf, Dr. Chem., Kreuzstrasse 6,
D-6710 Frankenthal (DE)**
Erfinder: **Schneider, Heinz-Walter, Dr. Chem.,
Bruesseler Ring 43, D-6700 Ludwigshafen (DE)**

## Verfahren zur Herstellung von Formylvaleriansäureestern

Gegenstand der Erfindung ist ein Verfahren zur Herstellung von Formylvaleriansäureestern, bei dem man Butadien oder Butadien enthaltende Kohlenwasserstoffgemische mit Kohlenmonoxid und Alkanolen in Gegenwart von tertiären Stickstoffbasen und Cobaltcarbonyl bei Temperaturen von 80 bis 150° C unter erhöhtem Druck umsetzt, und den so erhaltenen Pentensäureester mit Kohlenmonoxid und Wasserstoff bei Temperaturen von 100 bis 160° C unter erhöhtem Druck weiter zu Formylvaleriansäureestern umsetzt.

Aus der US-PS 3 253 018 ist bekannt, daß man zunächst Butadien mit Kohlenmonoxid und Methanol in Gegenwart eines Rhodiumkatalysators zu Pentensäuremethylestern umsetzt und diese aus dem Reaktionsgemisch isoliert. Die so erhaltenen Pentensäuremethylester werden dann in einer zweiten Stufe mit Kohlenmonoxid und Wasserstoff in Gegenwart eines Cobaltcarbonylkatalysators zu Formylvaleriansäuremethylester umgesetzt. Ein solches Verfahren ist aufwendig, da für jede Stufe ein gesonderter Katalysator verwendet werden muß und nach der ersten Stufe das Reaktionsgemisch quantitativ aufgearbeitet werden muß. Aus Bull. Chem. Soc. of Japan, Band 46, 1973, Seiten 524 bis 530 ist auch schon ein Verfahren zur Herstellung von Formylvaleriansäuremethylester bekannt, bei dem man in einer ersten Stufe Butadien, Kohlenmonoxid und Methanol in Gegenwart von Cobaltcarbonyl und Isochinolin bei einer Temperatur von 130° C und 300 bar zu Pentensäuremethylester umsetzt. Aus dem so erhaltenen Reaktionsgemisch wird Pentensäuremethylester abdestilliert und einer weiteren Stufe mit Kohlenmonoxid und Wasserstoff in Gegenwart von Cobaltcarbonyl bei einer Temperatur von 140 bis 200° C und einem Druck von 100 bis 250 bar zu Formylvaleriansäureester hydroformyliert. Will man den Katalysator aus der ersten Stufe auch in der zweiten Stufe verwenden, so müssen die mitverwendeten tertiären Stickstoffbasen abdestilliert werden. Hierbei zersetzt sich jedoch der Katalysator unter Ausscheidung von metallischem Cobalt und wird in seiner Wirksamkeit erheblich beeinträchtigt.

Es war deshalb die technische Aufgabe gestellt, ein Verfahren zur Herstellung von Formylvaleriansäurealkylestern zur Verfügung zu stellen, bei dem der Cobaltcarbonylkatalysator ohne Abzutrennen in beiden Stufen verwendbar ist.

Diese Aufgabe wird gelöst in einem Verfahren zur Herstellung von Formylvaleriansäurealkylester, wobei man in einer ersten Stufe Butadien oder solches enthaltende Kohlenwasserstoff-Gemische mit Kohlenmonoxid und Alkanolen in Gegenwart von Cobaltcarbonylkomplexen und 0,5 bis 2 Mol tertiäre Stickstoffbasen je Mol Butadien mit einem $pK_a$-Wert von 3 bis 11, bei einer Temperatur von 80 bis 150° C und unter einem Druck von 300 bis 2000 bar umsetzt und in einer zweiten Stufe die erhaltenen Pentensäurealkylester mit Kohlenmonoxid und Wasserstoff in Gegenwart von Cobaltcarbonylkomplexen bei einer Temperatur von 100 bis 160° C und unter einem Druck von 100 bis 300 bar umsetzt, wobei man aus den in der ersten Stufe erhaltenen Reaktionsgemisch tertiäre Stickstoffbasen überschüssige Alkanole und gegebenenfalls nicht umgesetzte Kohlenwasserstoffe abdestilliert, mit der Maßgabe, daß das Reaktionsgemisch vor oder während der Destillation mit molekularem Sauerstoff enthaltenen Gasen behandelt wird und daß das verbleibende Pentensäurealkylester und Cobaltkatalysator enthaltende Reaktionsgemisch in der zweiten Stufe verwendet.

Das neue Verfahren hat den Vorteil, daß bei den Verfahrensstufen nur ein Katalysator verwendet werden muß. Ferner hat das neue Verfahren den Vorteil, daß auf einfache und wirksame Weise die Zersetzung des Katalysators vermieden wird. Schließlich hat das neue Verfahren den Vorteil, daß es auf einfachere Weise kontinuierlich in technischem Maßstab geschaltet werden kann.

Man geht von reinen Butadien-1,3 oder Butadien enthaltenden Kohlenwasserstoff-Gemischen aus. Solche Kohlenwasserstoffgemische enthalten zum Beispiel neben Butadien gesättigte Kohlenwasserstoffe mit 3 bis 5 Kohlenstoffatomen und einfach ungesättigte Olefine mit 3 bis 5 Kohlenstoffatomen. Der Butadiengehalt soll in der Regel mehr als 10 Gewichtsprozent betragen. In der Technik werden insbesondere $C_4$-Schnitte als Ausgangsgemisch verwendet. Als solches seien alle Gemische von überwiegend unverzweigten $C_4$-Kohlenwasserstoffen definiert, die mehr als 10 Gew.-% Butadien und mehr als 15 Gew.-% Butene enthalten. Je nach Provenienz liegen die einzelnen Komponenten in solchen Gemischen normalerweise in folgenden Anteilen vor:

Butadien 40 bis 60 Gew.-%, Isobuten 20 bis 35 Gew.-%, Buten-1 10 bis 5 Gew.-%, Buten-2 5 bis 15 Gew.-%, Butane 1 bis 10 Gew.-%, Butine 0,1 bis 3 Gew.-%. Solche $C_4$-Schnitte fallen beispielsweise an bei der Dehydrierung von Butan oder Buten oder als Nebenprodukte bei der Äthylengewinnung durch thermische Spaltung (Cracken) von Leichtbenzin (Naphtha) oder höheren Kohlenwasserstoff-schnitten.

Bevorzugte Alkanole sind $C_1$- bis $C_4$-Alkanole wie Methanol, Äthanol, Propanol, Butanol oder Isobutanol. Besonders bevorzugt wird Methanol verwendet. Die Alkanole werden vorzugsweise im Überschuß angewandt, vorteilhaft von 1,5 bis 5 Mol je Mol Butadien.

Die Umsetzung führt man in der ersten Stufe bei Temperaturen von 80 bis 150° C, insbesondere 100 bis 140° C, und unter Drücken von 300 bis 2000 bar, insbesondere 600 bis 1200 bar, durch. Je Mol Butadien verwendet man in der Regel 0,01 bis 0,1 Grammatom Cobalt in Form von Cobaltcarbonylkomplexen.

Kohlenmonoxid wird im Überschuß vorteilhaft zum Beispiel dem 1,5 bis 10 stöchiometrischen erforderlichen Menge angewandt.

Geeignete tertiäre Stickstoffbasen mit einem $pK_a$-Wert von 3 bis 11 sind vorzugsweise N heterocyclische Verbindungen wie Pyridin ($pK_a$ 5,3) Methylpyridine wie 3-Picolin ($pK_a$ 6,0) Isochinolin ($pK_a$ 5,4), ferner Trialkylamine wie Trimethylamin ($pK_a$ 9,8) oder Triäthylamin ($pK_a$ 11,0). Vorteilhaft verwendet man tertiäre Stickstoffbasen, die niedriger sieden als der jeweils herzustellende Pentensäureester. Besondere technische Bedeutung hat Pyrodin erlangt. Vorteilhaft wendet man je Mol Cobaltcarbonylkatalysator 20 bis 50 Mol tertiäre Stickstoffbasen an.

Die in der ersten Stufe verwendeten Cobaltcarbonylkomplexe werden entweder in situ aus Cobaltsalzen, z. B. fettsauren Cobaltsalzen wie Formiat Acetat, oder Butyrat erzeugt. Vorteilhaft bringt man den Katalysator bereits als Cobaltcarbonyl ein. Insbesondere hat es sich bewährt, wenn man Cobaltcarbonylkatalysator gelöst in Butadien oder $C_4$-Schnitt in das Reaktionsgemisch einbringt. Eine solche Lösung erhält man beispielsweise wie folgt: Zunächst werden wäßrige Cobaltsalzlösungen mit Kohlenmonoxid und Wasserstoff im Überschuß bei Temperaturen von 50 bis 200° C und unter Drücken von 50 bis 500 bar in Gegenwart von Aktivkohle, die mit Cobaltcarbonyl geladen ist, behandelt. Bevorzugt verwendet man als Cobaltsalze Cobaltformiat oder Acetat. Zweckmäßig geht man von Lösungen aus, die 0,5 bis 5 Gew.-% Cobalt berechnet als Metall in Form der genannten Salze enthalten. Im allgemeinen enthält das genannte Gasgemisch Kohlenmonoxid und Wasserstoff im Volumenverhältnis 2 : 1 bis 1 : 1, insbesondere hat sich ein annähernd äquimolekulares Gewicht bewährt. Zweckmäßig wendet man das Gemisch aus Kohlenmonoxid und Wasserstoff in einem Überschuß z. B. bis zu dem Fünffachen der stöchiometrisch erforderlichen Menge an. Zum Beladen der Aktivkohle, mit Cobaltcarbonyl leitet man wäßrige Lösungen von Cobaltsalzen zusammen mit dem genannten Gasgemisch aus Kohlenmonoxid und Wasserstoff unter den angegebenen Reaktionsbedingungen über die Aktivkohle bis zu deren Sättigung, d. h. bis man im Ausgang Cobaltcarbonyl und Cobaltcarbonylwasserstoff analytisch feststellt.

Die so erhaltene Cobaltcarbonyl-Wasserstoff, nicht umgesetzte Cobaltsalze und freigesetzte Säure enthaltende wäßrige Lösung wird zweckmäßig zusammen mit dem nicht verbrauchten Gemisch aus Kohlenmonoxid und Wasserstoff vorteilhaft ohne zu entspannen mit Butadien oder Butadien enthaltenden Wasserstoffgemischen extrahiert. Es ist möglich, die gesamte für die Carbonylierung erforderliche Butadienmenge zur Extraktion zu verwenden oder nur einen Teil derselben, z. B. 5 bis 30 Mol Butadien pro Grammatom zu extrahierendes Cobalt. Die Extraktion erfolgt zweckmäßig in Gegenstrom oder Gleichstrom, beispielsweise in Kolonnen oder statischen Mischen bei Temperaturen von 20 bis 100° C und Drücken von 5 bis 300 bar. Das Gemisch wird anschließend in eine wäßrige Phase und eine organische Phase getrennt, wobei gleichzeitig das mitverwendete Gemisch aus Kohlenmonoxid und Wasserstoff als Gasphase abgetrennt wird. Das Cobaltgehalt der organischen Phase beträgt im allgemeinen von 1 bis 5 Gew.-%. Diese organische Phase wird als Katalysatorlösung für die Carbonylierung verwendet. Die abgetrennte wäßrige Phase enthält noch bis zu 1 Gew.-% Cobalt in Form des angewandten Salzes sowie bis zu 4 Gew.-% freie Säure, entsprechend dem angewandten Cobaltsalz. Diese wäßrige Lösung wird vorteilhaft wie später ausgeführt zur Wiedergewinnung des Cobaltkatalysators aus dem Reaktionsgemisch verwendet.

Das in der ersten Stufe (Carbonylierung) erhaltene Reaktionsgemisch enthält neben nicht umgesetztem Butadien gegebenenfalls andere Kohlenwasserstoffe tertiäre Stickstoffbasen, Cobaltcarbonylkatalysator, nicht umgesetzte Alkanole, die als Wertprodukte erzeugten Pentensäurealkylester sowie Nebenprodukte wie Valeriansäureester, Vinylcyclohexen, Butenyl- und Butylketonester sowie Polymerisate des Butadiens.

Aus dem vorgenannten Reaktionsgemisch werden erfindungsgemäß tertiäre Stickstoffbasen, überschüssige Alkanole und gegebenenfalls nicht umgesetzte Kohlenwasserstoffe abgetrennt, z. B. durch Destillation. Vorteilhaft erfolgt die Abtrennung quantitativ. Ein wesentliches Merkmal der Erfindung ist, daß das Reaktionsgemisch vor oder während der Abtrennung, d. h. Destillation mit molekularem Sauerstoff enthaltenden Gasen behandelt wird. Vorzugsweise wendet man die 1- bis 10fache äquivalente Menge an molekularem Sauerstoff je Grammatom Cobalt an. Molekularen Sauerstoff enthaltende Gase haben beispielsweise einen Gehalt an Sauerstoff von 0,5 bis 30 Vol.-% Sauerstoff. In der Technik hat sich Luft besonders bewährt. Vorteilhaft führt man die Behandlung mit molekularem Sauerstoff enthaltenden Gasen bei Temperaturen von 80 bis 100° C durch. Hierdurch wird es ermöglicht, die tertiären Stickstoffbasen quantitativ ohne die Zersetzung des Katalysators zu metallischem Cobalt aus dem Reaktionsgemisch zu entfernen. Führt man die Behandlung mit molekularen Sauerstoff enthaltenden Gasen während der Destillation durch, so leitet man diese zweckmäßig in den Kolonnensumpf ein. Wird die Behandlung vor der Destillation durchgeführt, behandelt man das Reaktionsgemisch beispielsweise 0,01 bis 5 Minuten mit molekularem Sauerstoff enthaltenden Gasen unter guter Durchmischung.

Der so erhaltene Destillationssumpf, der Pentensäurealkylester und Cobaltkatalysator und höhersiedende Nebenprodukte enthält, wird für die Hydroformulierung in der zweiten Stufe verwendet. Hierbei wird Pentensäurealkylester bei einer Temperatur von 100 bis 160°C und unter Drücken von 100 bis 300 bar mit Kohlenmonoxid und Wasserstoff in Gegenwart des im Reaktionsgemisch verbliebenen Cobaltkatalysators umgesetzt. Vorteilhaft hält man Temperaturen von 110 bis 130°C und Drücken von 50 bis 250 bar ein. Zusammensetzung und Menge des Gemisches aus Kohlenmonoxid und Wasserstoff entspricht demjenigen, wie der für die Katalysatorherstellung vorgenannt beschrieben wurde. Weiterhin hat es sich als vorteilhaft erwiesen, die Hydroformylierung unter der Mitverwendung von Lösungsmitteln durchzuführen. Geeignete Lösungsmittel sind beispielsweise Aromaten wie Benzol und Toluol, Äther wie Tetrahydrofuran oder Carbonsäureester wie Valeriansäureester, Buttersäureester oder Essigsäureester.

Nach dem Entspannen wird das erhaltene Reaktionsgemisch vorteilhaft mit Oxidationsmitteln wie Wasserstoffperoxid oder molekularem Sauerstoff enthaltenden Gasen, insbesondere Luft, behandelt. Die Oxidationsmittel wendet man vorteilhaft in einer Menge von 2 bis 10 Oxidationsäquivalenten je Mol Cobaltverbindung an. Die Behandlung erfolgt zweckmäßig unter Verwendung der sauren wäßrigen Lösung, die bei der Herstellung des Cobaltkatalysators anfällt. Der Lösung können zusätzlich geeignete Fettsäuren zugesetzt werden, um Cobalt in Lösung zu halten. Um die Cobaltlösung nicht in zu großer Verdünnung zu erhalten, ist es zweckmäßig, die wäßrige cobalthaltige Lösung im Kreis in den Behandlungsraum zurückzuführen und nur einen kleinen Teilstrom der dazugeführten Menge entsprechend abzutrennen. Die oxidative Behandlung erfolgt vorteilhaft bei Temperaturen von 80 bis 160°C, insbesondere 100 bis 130°C. Je nach dem Grad der Durchmischung ist die Umsetzung bereits nach wenigen Sekunden, vielfach bereits innerhalb von Bruchteilen einer Sekunden, beendet. Um eine gute Durchmischung zu gewährleisten, ist angezeigt, das Reaktionsgemisch unter gleichzeitiger Zuführung des Oxidationsmittels in feiner Verteilung der wäßrig sauren Lösung zuzuführen.

Nach der Behandlung trennt man das Gemisch, z. B. durch Dekantieren in eine organische Phase und eine wäßrige Phase. Cobaltsalze und gegebenenfalls die freie Säure enthaltende wäßrige Phase wird wieder für die Katalysatorherstellung verwendet. Aus der organischen Phase erhält man durch fraktionierte Destillation Lösungsmittel und nicht umgesetzte Pentensäureester, die wieder der Hydroformylierung zugeführt werden können sowie ein Gemisch aus Formylvaleriansäureester. Das Estergemisch eignet sich zur Herstellung von Diolen und Weichmachern. Aus dem Gemisch aus Formylvaleriansäureestern ist durch fraktionierte Destillation ω-Formylvaleriansäureester erhältlich, der sich zur Herstellung von ε-Caprolacton, ε-Capro-

lactam, Hexandiol sowie Adipinsäure eignet.

Das Verfahren nach der Erfindung sei an folgendem Beispiel veranschaulicht.

### Beispiel

Ein Hochdruckrohr, das mit 800 ml Aktivkohle gefüllt ist, wird stündlich mit 400 ml einer wäßrigen bezüglich Cobalt 2%igen Cobaltacetatlösung (bezogen auf Cobalt) beschickt. Außerdem führt man stündlich 110 Nl eines äquimolaren Gemisches aus Kohlenmonoxid und Wasserstoff zu. Hierbei hält man eine Temperatur von 140°C und einen Druck von 300 bar ein. Die im anderen Teil des Rohres abgezogene Lösung enthält 0,5 Gew.-% $Co^{2+}$ und 1,5 Gew.-% Co als Cobaltcarbonylwasserstoff und die entsprechende Menge Essigsäure. Diese Lösung wird nach dem Entspannen auf 20 bar bei 40°C intensiv mit 540 ml eines $C_4$-Schnittes, der 40 Gew.-% Butadien (2,52 Mol) enthält, gemischt. Nach der Phasentrennung enthält der $C_4$-Schnitt 6 g Cobalt in Form von Cobaltcarbonylverbindungen. Dieser Cobaltcarbonylverbindungen enthaltende $C_4$-Schnitt wird nun einem Hochdruckgefäß von 2,5 l Inhalt zugeführt und außerdem werden stündlich 200 ml (2,52 Mol) Pyridin und 200 ml (5,04 Mol) Methanol und 120 Nl Kohlenmonoxid zugegeben. Die Carbonylierung verläuft bei einer Temperatur von 135°C und 900 bar. Das im Kopf des Hochdruckgefäßes abgenommene Produkt wird entspannt, wobei gasförmig neben überschüssigem Kohlenmonoxid überschüssige $C_4$-Kohlenwasserstoffe, die nahezu Butadienfrei sind, abgetrennt werden. Danach wird das Pyridin und das nicht umgesetzte Methanol (300 ml Destillat/h) aus dem Reaktionsaustrag abdestilliert, wobei bei einer Temperatur von 80°C pro Stunde 50 Nl dem Kolonnensumpf zugeführt werden. Der Destillationssumpf, der 6 g Cobaltkatalysator (berechnet als Kobalt) und 258 g (2,27 Mol) Pentensäureester enthält, wird nach Zugabe von 258 g Buttersäureester als Verdünnungsmittel zusammen mit 100 Nl Synthesegas ($CO:H_2 = 1:1$) kontinuierlich einem weiteren Hochdruckgefäß von 2,4 l Volumen von unten zugeführt. Die Hydroformylierung wird bei einer Temperatur von 120°C und einem Druck von 200 bar durchgeführt. Eine gaschromatographische Analyse des anfallenden Austrages (580 g/h zeigt, daß von dem zugeführten Pentensäureester 2% zu Valeriansäureester, 93% zu Formylvaleriansäureestern mit einem Normalanteil von 70% und 5% zu Hochsiedern (Aldolisierungsprodukte) umgesetzt sind.

Der Reaktionsaustrag wird mit 400 ml/h der in der Extraktionsstufe anfallenden essigsauren wäßrigen Lösung unter Durchleiten von 300 Nl/h Luft bei 100°C in einem mit Raschigringen gefüllten Rohr gut durchmischt. Nach der Trennung werden 400 ml wäßrige 2%ige Cobaltacetatlösung erhalten, die in die Stufe a) zur Vorcarbonylierung zurückgeführt werden. Die organische Phase wird durch fraktionierte Destillation in Buttersäureester (ca. 250 g, Valeriansäureester (ca. 5 g), Formylvaleriansäureester (ca. 300 g) und Hochsieder (ca. 20 g) aufgetrennt.

## Patentansprüche

1. Verfahren zur Herstellung von Formylvaleriansäurealkylestern, wobei man in einer ersten Stufe Butadien oder solches enthaltende Kohlenwasserstoffgemische mit Kohlenmonoxid und Alkanolen in Gegenwart von Cobaltcarbonylkomplexen und 0,5 bis 2 Mol tertiären Stickstoffbasen je Mol Butadien mit einem $pK_a$-Wert von 3 bis 11 bei einer Temperatur von 80 bis 150°C und unter einem Druck von 300 bis 2000 bar umsetzt und in einer zweiten Stufe die erhaltenen Pentensäurealkylester mit Kohlenmonoxid und Wasserstoff in Gegenwart von Cobaltcarbonylkomplexen bei einer Temperatur von 100 bis 160°C unter einem Druck von 100 bis 300 bar umsetzt, dadurch gekennzeichnet, daß man aus dem in der ersten Stufe erhaltenen Reaktionsgemisch tertiäre Stickstoffbasen, überschüssige Alkanole und gegebenenfalls nicht umgesetzte Kohlenwasserstoffe abdestilliert mit der Maßgabe, daß das Reaktionsgemisch vor oder während der Destillation mit molekularem Sauerstoff enthaltenden Gasen behandelt wird und das verbleibende Pentensäurealkylester und Cobaltkatalysator enthaltende Reaktionsgemisch in der zweiten Stufe verwendet.

2. Verfahren nach Anspruch 1, dadurch gekennzeichnet, daß man die Behandlung mit molekularem Sauerstoff enthaltenden Gas bei einer Temperatur von 80 bis 100°C durchführt.

3. Verfahren nach den Ansprüchen 1 und 2, dadurch gekennzeichnet, daß man je Grammatom Cobalt 1 bis 10 Oxidationsäquivalente an molekularem Sauerstoff anwendet.

4. Verfahren nach den Ansprüchen 1 bis 3, dadurch gekennzeichnet, daß man das aus der zweiten Stufe erhaltene Reaktionsgemisch mit der bei der Herstellung des Cobaltcarbonylkatalysators anfallenden sauren wäßrigen Lösung und molekularen Sauerstoff enthaltenden Gasen bei einer Temperatur von 80 bis 160°C behandelt und nach Abtrennung der organischen Phase die Cobaltsalze enthaltende wäßrige Phase wieder für die Herstellung des Cobaltcarbonylkatalysators verwendet.

## Claims

1. A process for the preparation of alkyl formylvalerates, in which butadiene or a butadiene-containing hydrocarbon mixture is reacted, in a first stage, with carbon monoxide and alkanols in the presence of cobalt carbonyl complexes and, per mole of butadiene, from 0.5 to 2 moles of tertiary nitrogen bases having a $pK_a$ of from 3 to 11, at from 80 to 150°C and from 300 to 2,000 bar, and, in a second stage, the resulting alkyl pentenoate is reacted with carbon monoxide and hydrogen in the presence of cobalt carbonyl complexes at from 100 to 160°C and from 100 to 300 bar, characterized in that tertiary nitrogen bases, excess alkanols and any unconverted hydrocarbons are distilled off from the reaction mixture obtained in the first stage, with the proviso that the reaction mixture is treated, before or during the distillation, with gases containing molecular oxygen, and the residual reaction mixture containing alkyl pentenoate and cobalt catalyst is used in the second stage.

2. A process as claimed in claim 1, characterized in that the treatment with gas containing molecular oxygen ist carried out at from 80 to 100°C.

3. A process as claimed in claims 1 and 2, characterized in that 1 — 10 oxidation equivalents of molecular oxygen per gram atom of cobalt are used.

4. A process as claimed in claims 1 to 3, characterized in that the reaction mixture obtained from the second stage ist treated with the acidic aqueous solution obtained from the preparation of the cobalt carbonyl catalyst and with gases containing molecular oxygen at from 80 to 160°C and, after separating off the organic phase, the aqueous phase containing the cobalt salt is re-used for preparation of the cobalt carbonyl catalyst.

## Revendications

1. Procédé de préparation d'esters alkyliques d'acide formylvalérianique, où, dans un premier stade, on fait réagir, à une température de 80 à 150°C et sous une pression de 300 à 2000 bars, du butadiène, ou mélange d'hydrocarbures en contenant avec de l'oxyde de carbone et des alcanols, en présence de complexes cobalt-carbonyle et par mole de butadiène 0,5 à 2 moles de bases azotées tertiaires, d'une valeur $pK_a$ de 3 à 11 et, dans un second stade, on fait réagir l'ester alkylique d'acide penténique avec de l'oxyde de carbone et de l'hydrogène en présence de complexes cobalt-carbonyle, à une température de 100 à 160°C, sous une pression de 100 à 300 bars, caractérisé par le fait que, du mélange de réaction obtenu dans le premier stade, on sépare, par distillation, des bases azotées des alcanols en excès et éventuellement des hydrocarbures n'ayant pas réagi, sous réserve qu'on traite le mélange de réaction, avant ou pendant la distillation, avec des gaz contenant de l'oxygène moléculaire et qu'on utilise dans le deuxième stade le mélange de réaction contenant l'ester alkylique d'acide penténique et le catalyseur de cobalt restants.

2. Procédé selon la revendication 1, caractérisé par le fait qu'on effectue le traitement avec du gaz contenant de l'oxygène moléculaire à une température de 80 à 100°C.

3. Procédé selon l'une des revendications 1 et 2, caractérisé par le fait qu'on utilise, par atome-gramme de cobalt, 1à 10 équivalents d'oxydation en oxygène moléculaire.

4. Procédé selon l'une des revendications 1 à 3, caractérisé par le fait qu'on traite, à une température de 80 à 160°C, le mélange de réaction, obtenu au second stade, avec la solution aqueuse acide, formé lors de la préparation du catalyseur cobalt-carbonyle et les gaz contenant de l'oxygène moléculaire et, après séparation de la phase organique, on réutilise la phase aqueuse contenant des sels de cobalt pour la préparation du catalyseur cobalt-carbonyle.